# EUROPÄISCHE PATENTSCHRIFT

(11) **EP 1 046 391 B1**
(45) Veröffentlichungstag und Bekanntmachung des Hinweises auf die Patenterteilung: **15.09.2004**
(21) Anmeldenummer: 00105806.4
(22) Anmeldetag: 18.03.2000
(51) Int. Cl.: A61K 7/42, C07C 225/22, C07D 295/10

(54) **Verwendung von aminosubstituierten Hydroxybenzophenonen als photostabile UV-Filter in kosmetischen und pharmazeutischen Zubereitungen**
Use of aminosubstituted hydroxybenzophenones as photostable UV-filters in cosmetic and pharmaceutical preparations
Usage d'hydroxybenzophénones aminosubstituées comme filtres UV photostables dans des préparations cosmétiques et pharmaceutiques

(30) Priorität: 20.04.1999 DE 19917906
(43) Veröffentlichungstag der Anmeldung: 25.10.2000
(60) Teilanmeldung: 04009762.8
(73) Patentinhaber: BASF AKTIENGESELLSCHAFT, 67056 Ludwigshafen (DE)
(72) Erfinder: Habeck, Thorsten, Dr., 67149 Meckenheim (DE); Prechtl, Frank, Dr., 60318 Frankfurt (DE); Wünsch, Thomas, Dr., 67346 Speyer (DE); Westenfelder, Horst, 67435 Neustadt (DE); Haremza, Sylke, Dr., 69151 Neckargemünd (DE); Bach, Thorsten, Prof. Dr., 35039 Marburg (DE); Spiegel, Anja, 35037 Marburg (DE)

(56) Entgegenhaltungen:
- PATENT ABSTRACTS OF JAPAN vol. 012, no. 391 (C-537), 18. Oktober 1988 (1988-10-18) & JP 63 139158 A (KANEBO LTD), 10. Juni 1988 (1988-06-10)
- W.R. BROWN ET AL.: "The blue sodium salt of rhodamine-b and some related substances" JOURNAL OF THE CHEMICAL SOCIETY, 1933, Seiten 1264-1269, XP000939380 LETCHWORTH GB
- PATENT ABSTRACTS OF JAPAN vol. 017, no. 567 (C-1120), 14. Oktober 1993 (1993-10-14) & JP 05 163275 A (MITSUI TOATSU CHEM INC), 29. Juni 1993 (1993-06-29)

## Beschreibung

Die Erfindung betrifft die Verwendung von aminosubstituierten-Hydroxybenzophenonen als photostabile UV-A-Filter in kosmetischen und pharmazeutischen Zubereitungen zum Schutz der menschlichen Epidermis oder menschlichen Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, die Lichtschutzmittel erfüllen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PAR-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 und EP-A-0 416 837 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

JP 63 139 158 beschreibt u.a. aminosubstituierte Hydroxybenzephenone als UV-Filter in kosmetischen Zusammensetzungen. Die Photostabilität bzw. die Absorptionseigenschaften der hier offenbarten Verbindungen sind für kosmetische Anwendungen nicht oder nur unzureichend gewährleistet

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe

Eigenfarbe d.h. eine scharfe Bandenstrukur aufweisen und je nach Substituent in Öl oder Wasser löslich sind.

Diese Aufgabe wurde erfindungsgemäß gelöst durch Verwendung von aminosubstituierten Hydroxybenzophenonen der allgemeinen Formel Ib 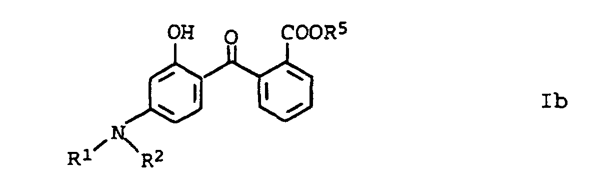 in der die Substituenten unabhängig voneinander folgende Bedeutung haben:
- R¹ und R²: Wasserstoff, C₁-C₁₂-Alkyl, wobei die Substituenten R¹ und R² gemeinsam mit dem Stickstoffatom, an dem sie gebunden sind, einen 5- oder 6-Ring bilden können;
- R⁵: Wasserstoff, C₁-C₁₂-Alkyl, C₃-C₆-Cycloalkyl.
als photostabile UV-A-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im Bereich absorbierenden Verbindungen.

Als Alkylreste R¹, R² und R⁵ seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder 2-Ethylhexyl genannt.

Als besonders bevorzugte Alkylreste seien für R¹, R² und R⁵ Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.

Als C₃-C₆-Cycloalkylreste seien für R⁵ besonders bevorzugt Cyclopropyl, Cyclopentyl und Cyclohexyl genannt.

Weiterhin weisen Verbindungen der Formel Ib besondere photostabile Eigenschaften aus, bei denen die Substituenten R¹, R² und R⁵ in der in Tabelle 1 genannten Kombination vorliegen.

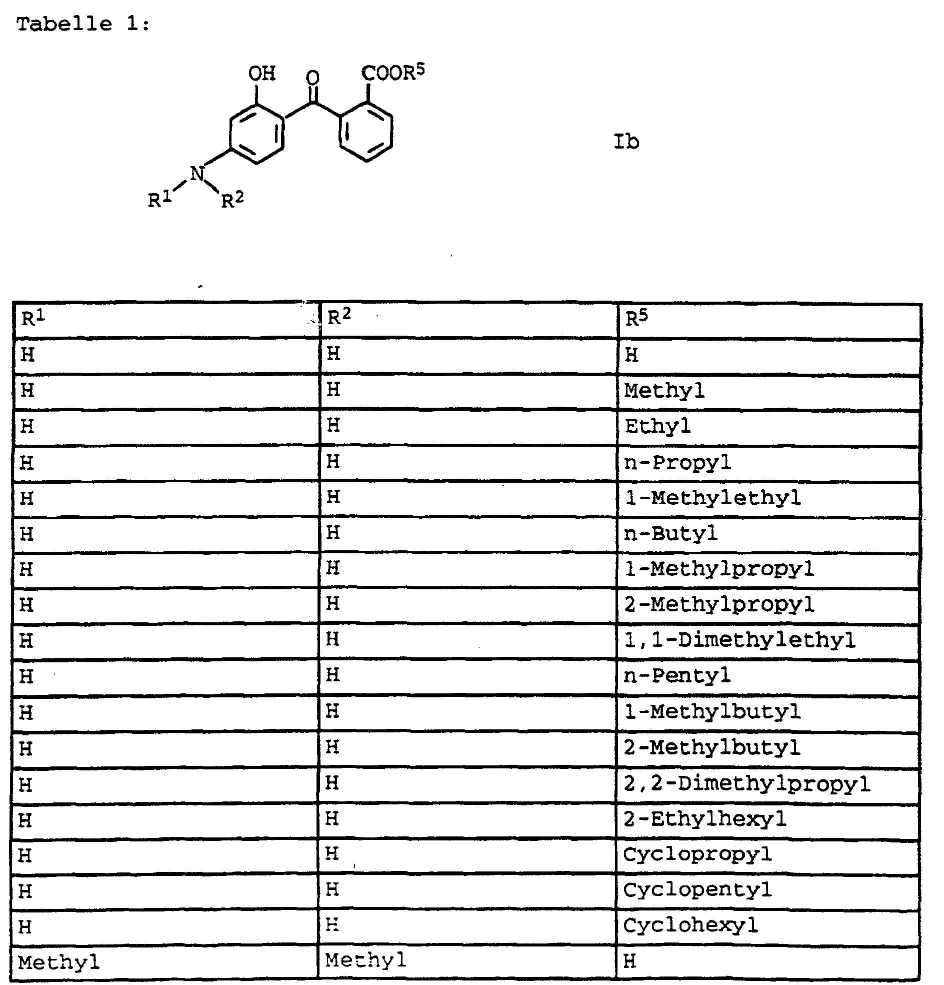

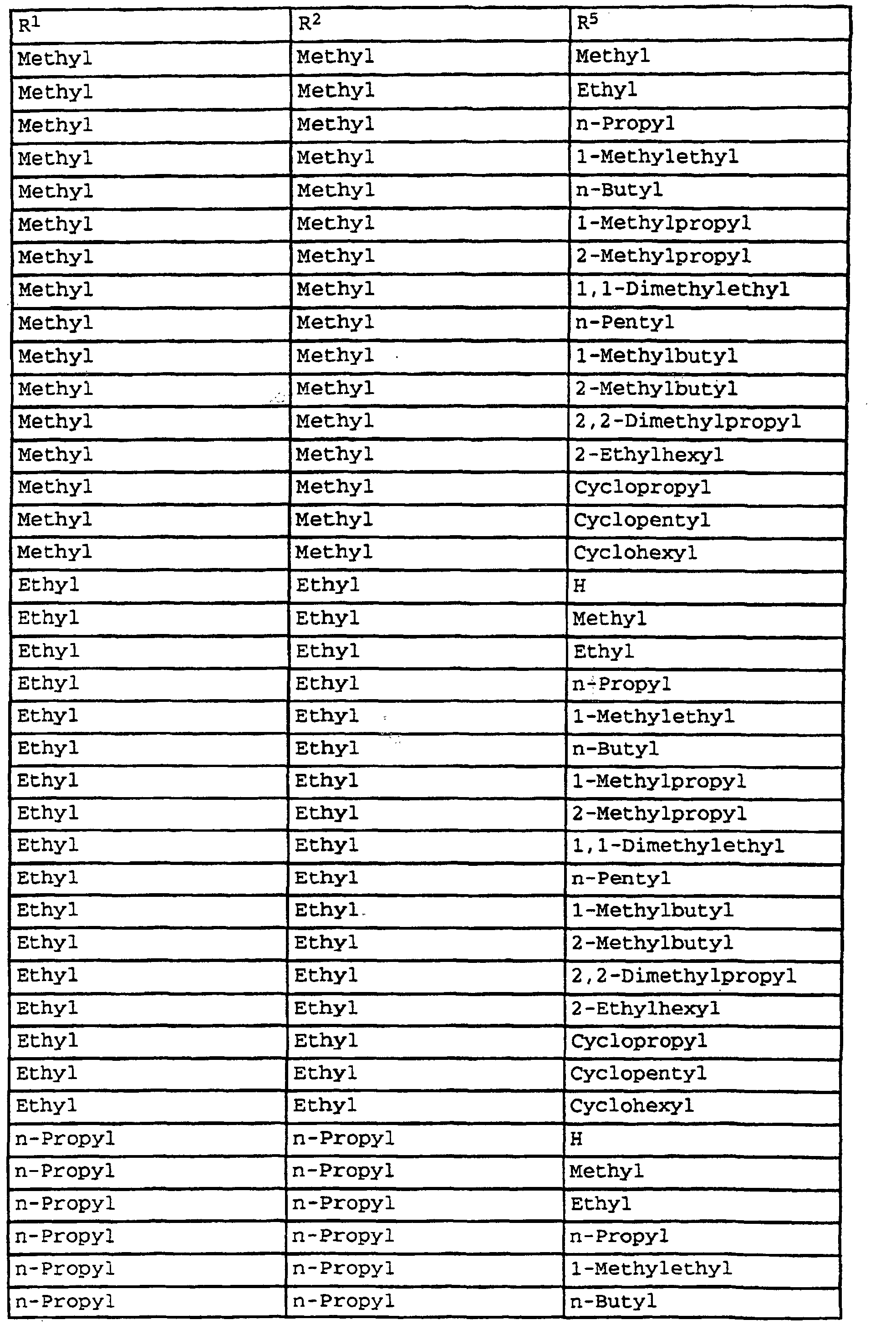

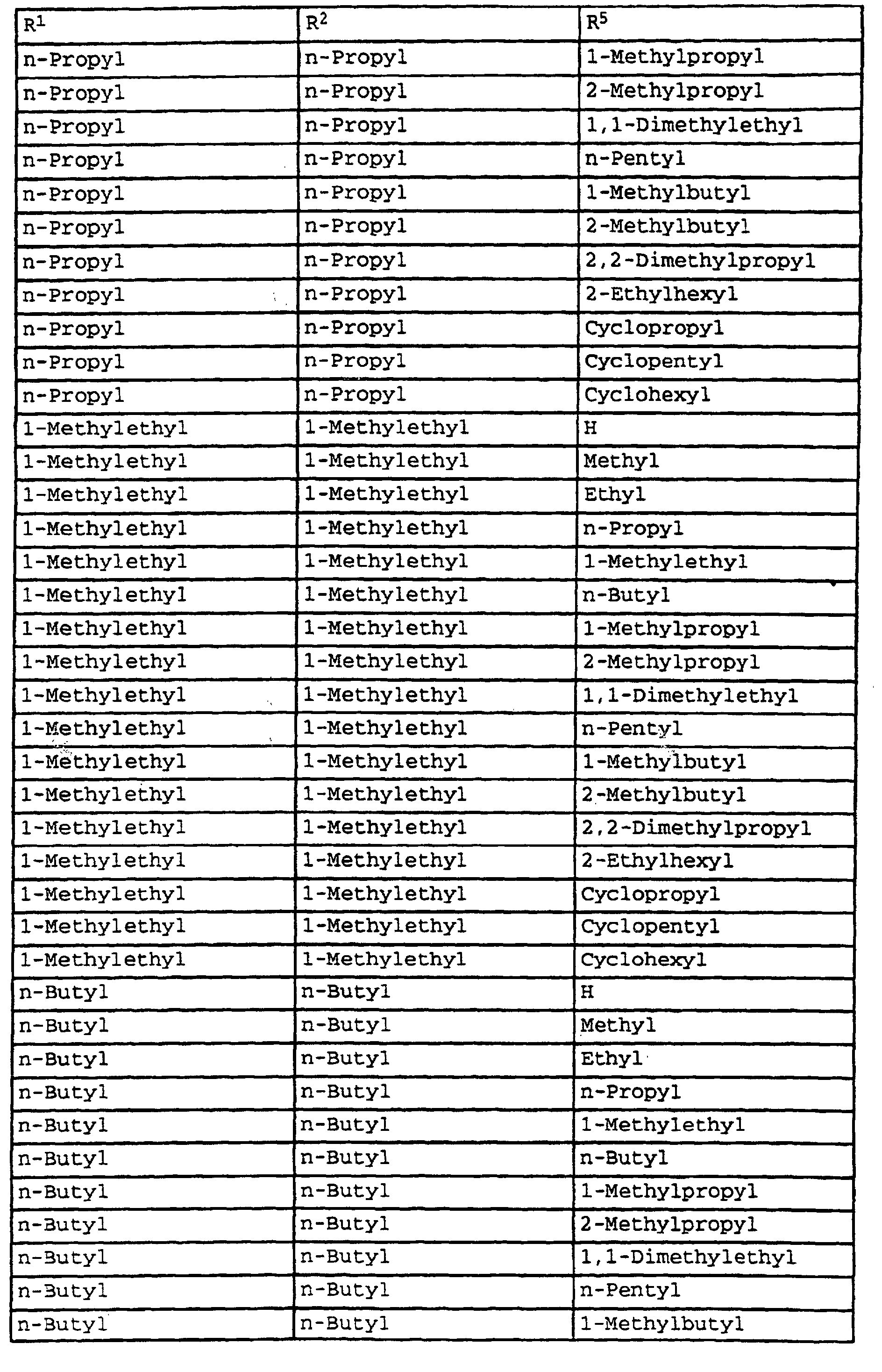

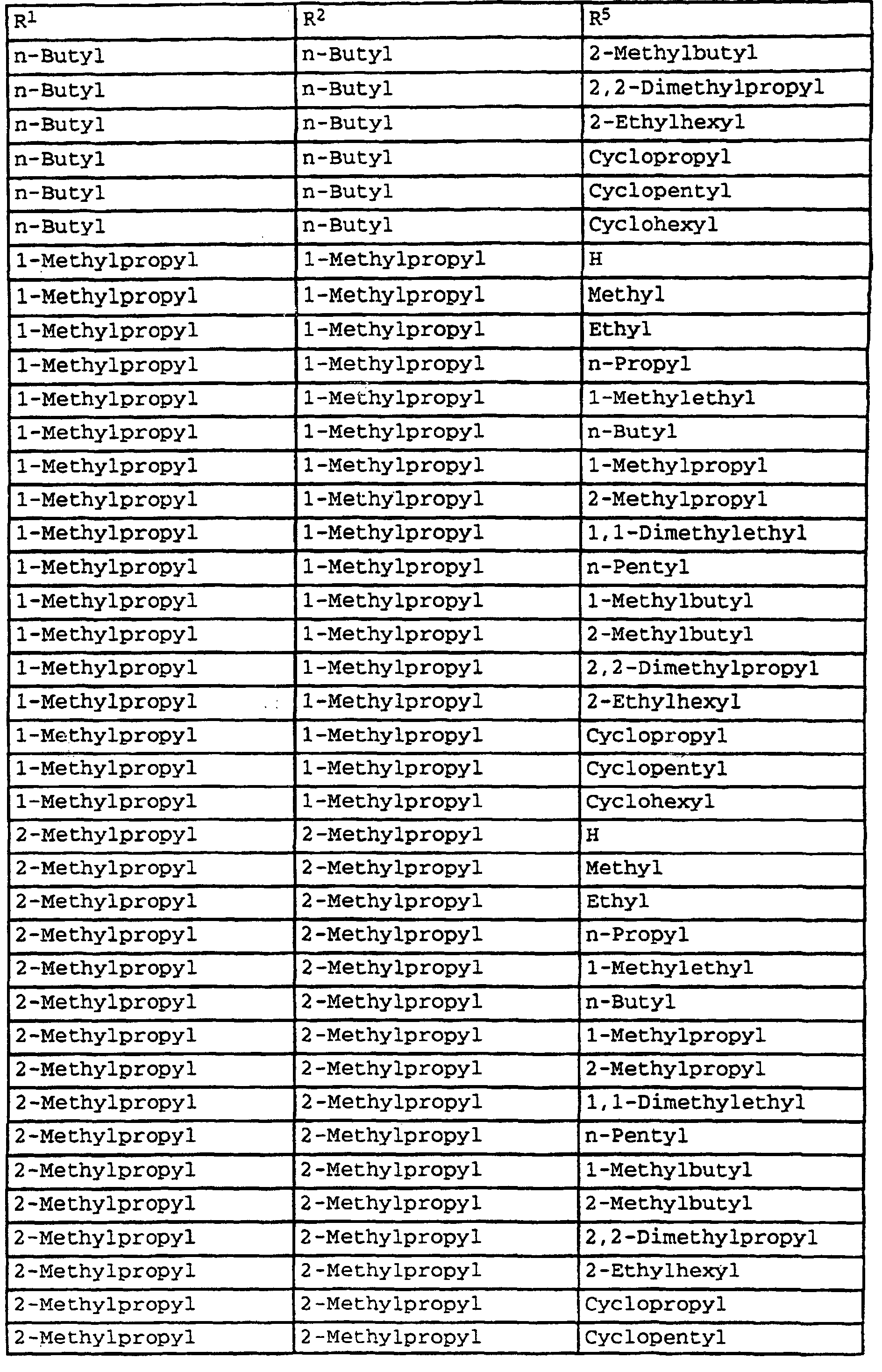

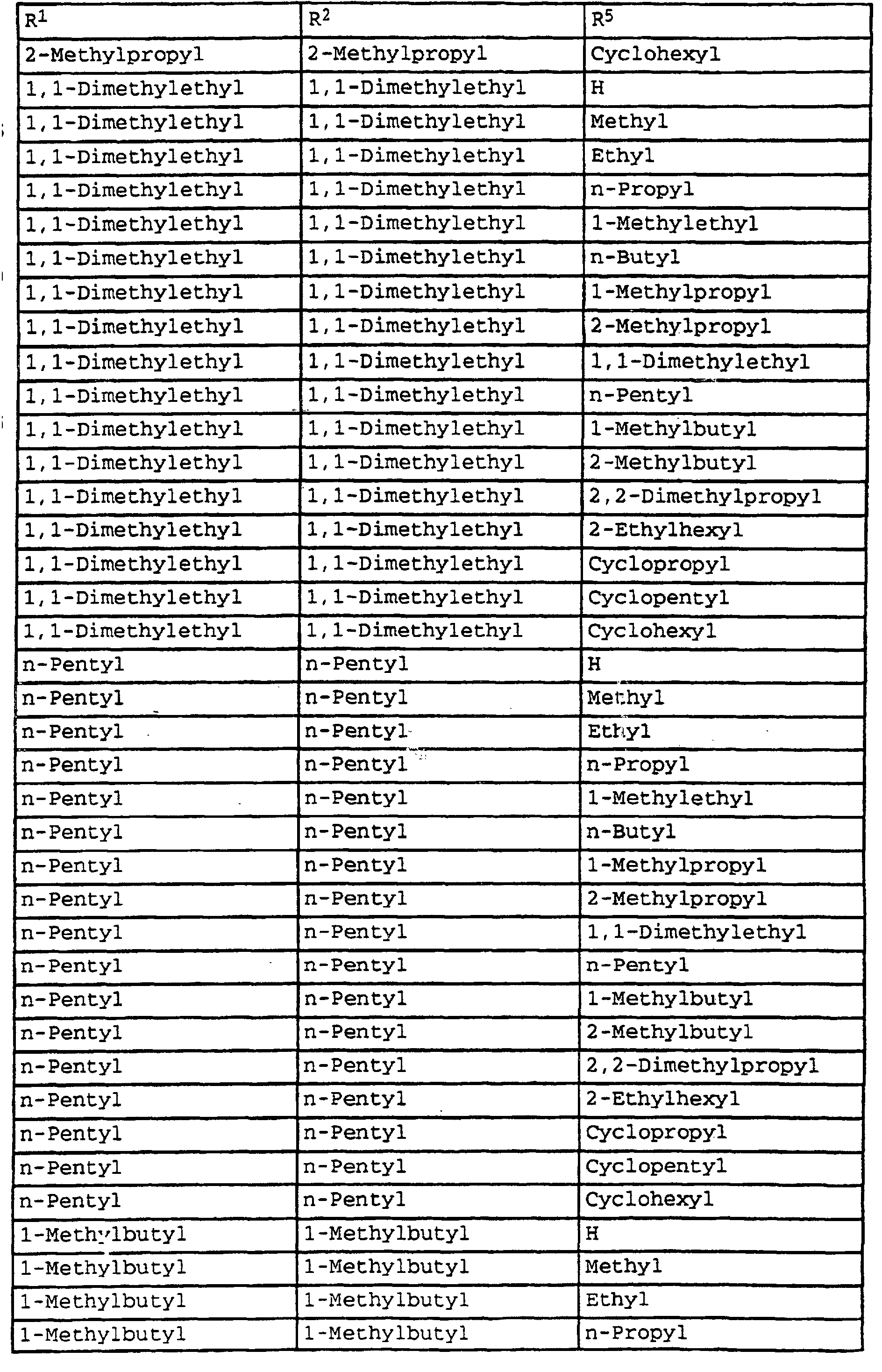

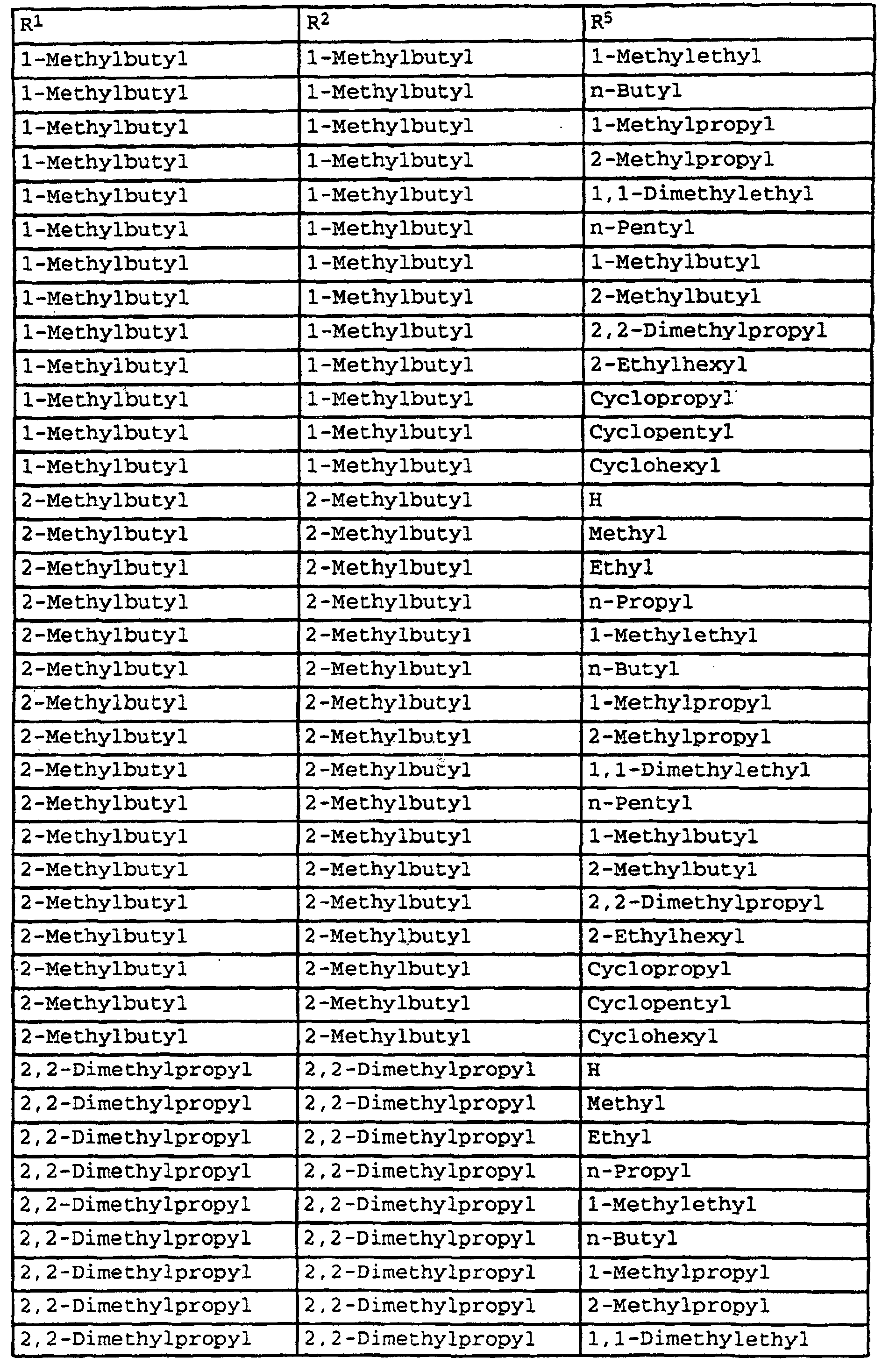

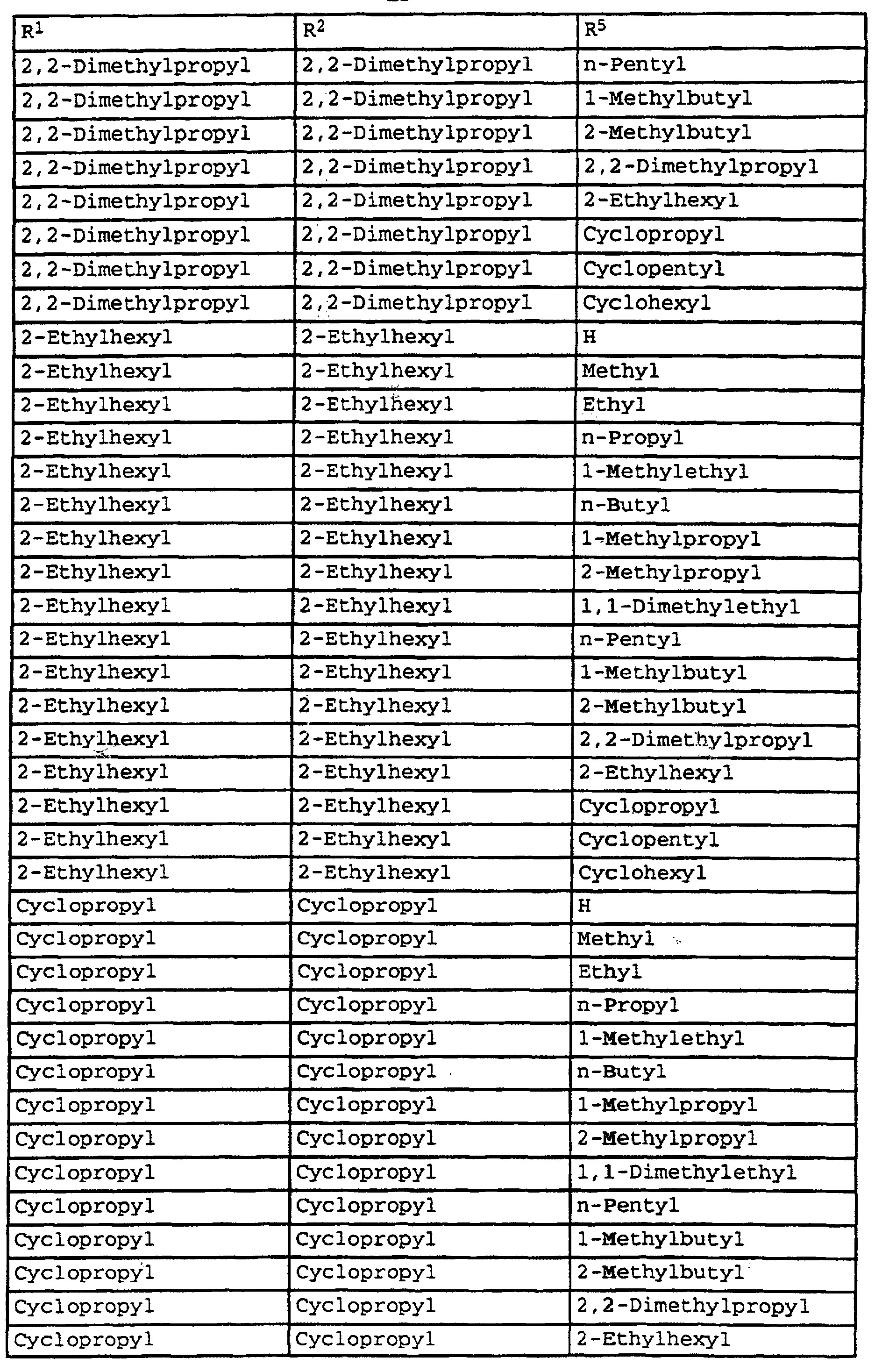

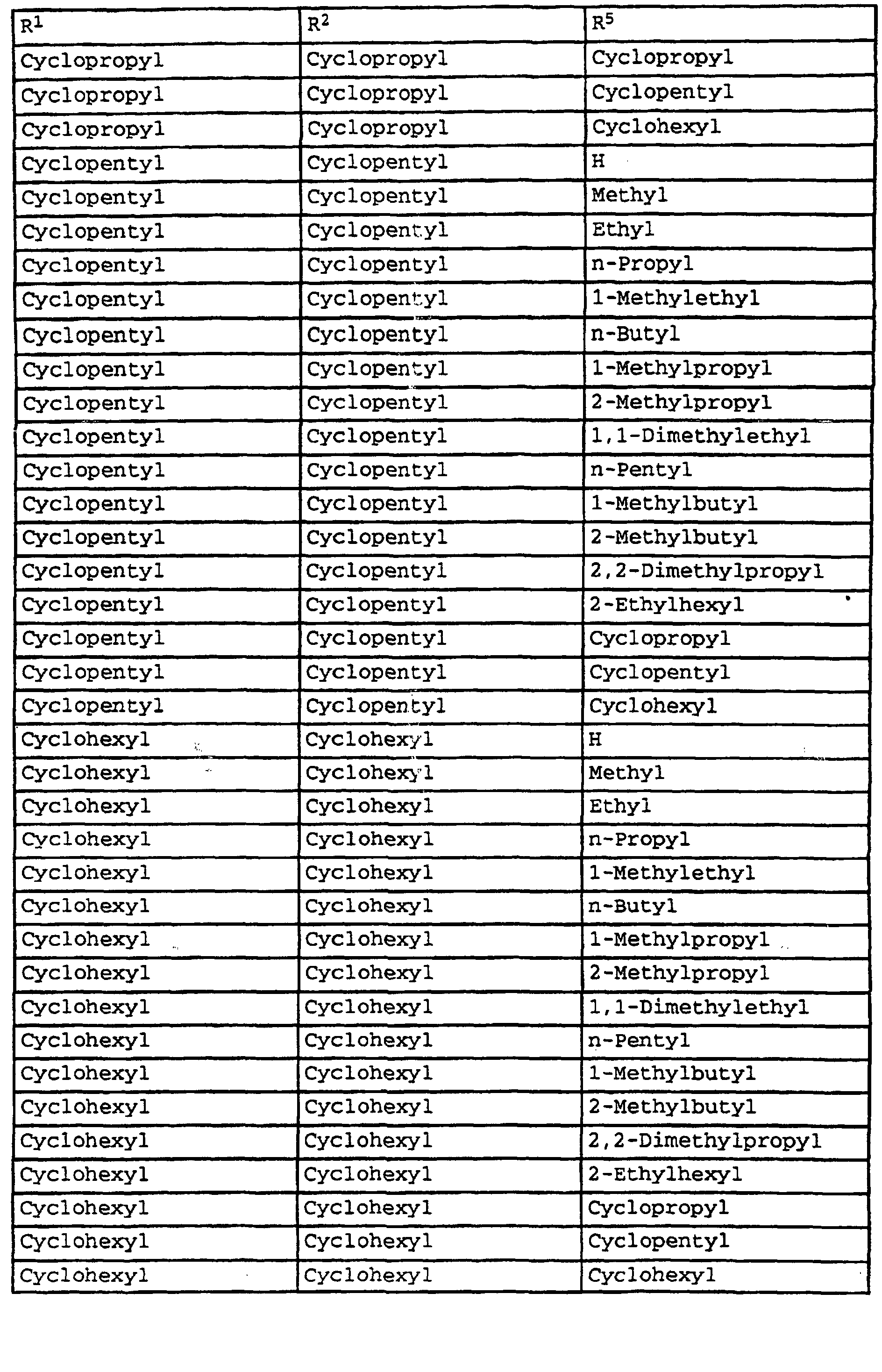

Die in Tabelle I genannten Verbindungen zeigen eine Photostabilität von > 95%, bevorzugt > 98% auf.

Die Erfindung betrifft auch aminosubstituierte Hydroxybenzophenone der Formel Ic, 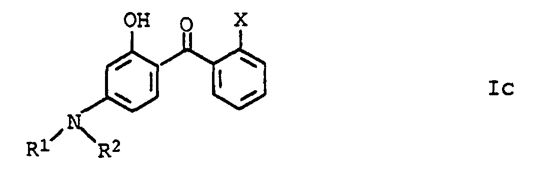 in der die Variablen unabhängig voneinander folgende Bedeutung haben:
- R¹ und R²: Wasserstoff, C₁-C₈-Alkyl, wobei die Substituenten R¹ und R² gemeinsam mit dem Stickstoffatom, an dem sie gebunden sind, einen 5- oder 6-Ring bilden können;
- X: COOR⁵, CONR⁶R⁷;
- R⁵: C₂-C₁₂-Alkyl, C₅-C₆-Cycloalkyl;
- R⁶ und R⁷: Wasserstoff, C₁-C₁₂-Alkyl, C₅-C₆-Cycloalkyl.

Als Alkylreste R¹ und R² seien verzweigte oder unverzweigte C₁-C₈-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder 2-Ethylhexyl genannt.

Als Alkylreste R⁵ seien verzweigte oder unverzweigte C₂-C₁₂-Alkylketten, bevorzugt Ethyl, n-Propyl, 1-Methylethyl, n-Butyl, 1-Methylpropyl-, 2-Methylpropyl, 1, 1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, 2-Ethylhexyl, n-Nonyl, n-Decyl, n-Undecyl oder n-Dodecyl genannt.

Als Alkylreste R⁶ und R⁷ seien verzweigte oder unverzweigte C₁-C₁₂-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl oder 2-Ethylhexyl genannt.

Als Cycloalkylreste seien für R⁵ bis R⁷ bevorzugt verzweigte oder unverzweigte C₅-C₆-Cycloalkylketten wie Cyclopentyl oder Cyclohexyl genannt.

Bevorzugt sind Verbindungen der Formel Ic, in der unabhängig voneinander R¹ und R² C₁-C₄-Alkyl, R⁵ C₃-C₈-Alkyl und R⁶ und R⁷ C₁-C₈-Alkyl bedeuten.

Besonders bevorzugt sind Verbindungen der Formel Ic, in der unabhängig voneinander R¹ und R² Ethyl, R⁵ C₅-C₈-Alkyl und R⁶ und R⁷ C₁-C₈-Alkyl aus den jeweils oben vorliegenden Auflistungen der Substituenten bedeuten.

Die erfindungsgemäß zu verwendenden Verbindungen der Formel I können durch direkte Acylierung der entsprechenden aminosubstituierten Phenole hergestellt werden.

So kann beispielsweise die Synthese von 2-(4-Dimethylamino-2-hydroxylbenzoyl)-benzoesäuremethylester (1) bzw. 2-(2-Hydroxy-4-pyrrolidin-1-yl-benzoyl)-benzoesäuremethylester (2) durch Acylierung von meta-Diethylaminophenol bzw. 3-Pyrrolidin-1-yl-phenol mit Phthalsäureanhydrid und anschließender Veresterung erfolgen.

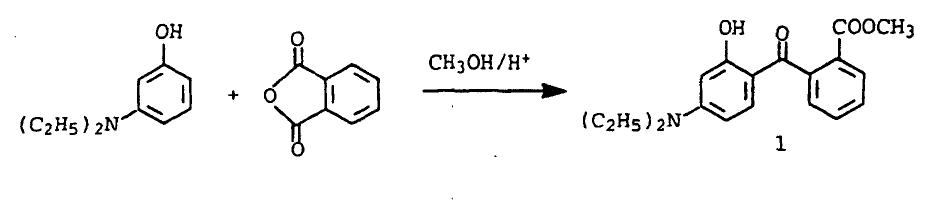

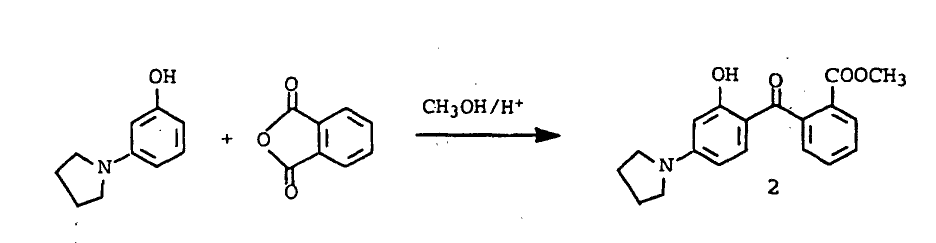

Ferner läßt sich (4-Diethylamino-2-hydroxylbenzoyl)-phenylmethanon (3) beispielsweise durch Umsetzung von meta-Diethylaminophenol mit Benzoylchlorid und anschließender Fries-Umlagerung mit AlCl₃ herstellen.

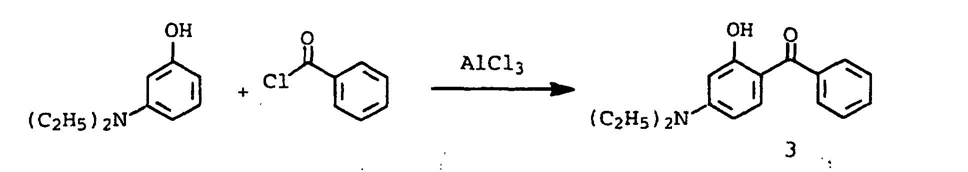

Einzelheiten zur Friedel-Crafts Reaktion sowie zur Fries'schen Verschiebung finden sich im Organikum, VEB Deutscher Verlag der Wissenschaften, Berlin, 1986, S. 323-327 sowie im Houben-Weyl, Bd. 7/2a, 1973, S. 379-389.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel Ib zusammen mit an sich für kosmetische und pharmazeutische Zubereitungen bekannten, im UV-A- und UV-B-Bereich absorbierenden Verbindungen als Lichtschutzmittel enthalten, wobei die Verbindungen der Formel Ib in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Als Emulsionen kommen u.a. auch O/W-Makroemulsionen, O/W-Mikroemulsionen oder O/W/O-Emulsionen mit in dispergierter Form vorliegenden aminosubstituierten Hydroxybenzophenonen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure lensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnehschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus W-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Verbindungen der Formel Ib angewandt werden, kommen beliebige UV-A- und UV-B-Filtersubstanzen in Betracht. Beispielsweise sind zu nennen:

| Nr. | Stoff | CAS-Nr. ( =Säure) |
|---|---|---|
| 1 | 4 -Aminobenzoesäure | 150-13-0 |
| 2 | 3- (4'Trimethylammonium) -benzylidenbornan-2-on-methylsulfat | 52793-97-2 |
| 3 | 3, 3, 5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis (7,7-dimethyl-2 -oxobicyclo [2.2.1] heptan-1-methansulfonsäure) und ihre Salze | 90457-82-2 |
| 7 | 4-Bis (polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4, 6-Trianilin-(o-carbo-2'-ethylhexyl-1'-oxy)-1,3, 5-triazin | 88122-99-0 |
| 18 | 3-Imidazol-4-yl-acrylsäure und ihr Ethylester | 104-98-3 |
| 19 | 2-Cyano-3, 3-diphenylacrylsäureethylester | 5232-99-5 |
| 20 | 2-Cyano-3, 3-diphenylacrylsäure-2'-ethylhexylester | 6197-30-4 |
| 21 | Menthyl-o-aminobenzoate oder: 5-Methyl-2- (1-methylethyl) -2-aminobenzoate | 134-09-8 |
| 22 | Glyceryl p-aminobenzoat oder: 4-Aminobenzoesäure-1 -glyceryl-ester | 136-44-7 |
| 23 | 2,2' -Dihydroxy-4-methoxybenzophenon (Dioxybenzone) | 131-53 -3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3, 4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4 '-methoxy-dibenzoylmethan | 70356-09-1 |
| 29 | 2,2', 4,4 '-Tetrahydroxybenzophenon | 131-55-5 |
| 30 | 2,2'-Methylen-bis-[6(2H-benzotriazol-2-y1)-4-(1,1,3,3,-tetramethylbutyl)phenol] | 103597-45-1 |
| 31 | 2,2'-(1,4-Phenylen)-bis-1H-benzimidazol-4,6-disulfonsäure, Na-Salz | 180898-37-7 |
| 32 | 2,4-bis-[4-(2-Ethylhexyloxy)-2-hydroxy]phenyl-6-(4-methoxyphenyl)-(1,3,5)-triazin | 187393-00-6 |

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindugsgemäßen Lichtschutzmittel der Formel Ib in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den Verbindungen Ib hergestellten Emulsionen zeichnen sich besonders durch ihre hohe Stabilität, die Verbindungen Ib selber durch ihre hohe Photostabilität aus, und die mit Ib hergestellten Zubereitungen durch ihr angenehmes Hautgefühl aus.

Die UV-Filterwirkung der erfindungsgemäßen Verbindungen der Formel Ib kann auch zur Stabilisierung von Wirk- und Hilfsstoffen in kosmetischen und pharmazeutischen Formulierungen ausgenutzt werden.

In den folgenden Beispielen wird die Herstellung und Verwendung der aminosubstituierten Hydroxybenzophenone näher erläutert.

### Beispiele:

### I. Herstellung

### Beispiel 1

### Herstellung von 2-(4-Diethylamino-2-hydroxybenzoyl)-benzoesäuremethylester

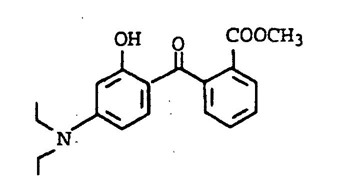

Eine Lösung von 3,11 g (18,8 mmol) 3-Diethylaminophenol und 2,88 g (19,4 mmol) Phthalsäureanhydrid in 60 ml abs. Toluol wurde 21 h unter Rückfluß erhitzt. Nach Entfernen des Lösungsmittels im Vakuum wurde der Rückstand in 100 ml Chloroform gelöst. Die organische Phase wurde je sechsmal mit 20 ml verdünnter Salzsäure und einmal mit 20 ml Wasser gewaschen, dann sechsmal mit je 20 ml gesättigter Natriumhydrogencarbonatlösung extrahiert. Die vereinigten wäßrigen Extrakte wurden mit verd. Schwefelsäure auf pH < 2 angesäuert und dreimal mit 50 ml Diethylether extrahiert. Die vereinigten Etherextrakte wurden dreimal mit je 50 ml Wasser gewaschen und dreimal mit je 50 ml gesättigter Natriumhydrogencarbonatlösung extrahiert. Die vereinigten wäßrigen Extrakte wurden auf pH < 2 angesäuert und dreimal mit 50 ml Diethylether extrahiert. Die organischen Phasen wurden vereinigt, mit 50 ml Wasser gewaschen, über Magnesiumsulfat getrocknet, filtriert und das Lösungsmittel im Vakuum entfernt.

Das Rohprodukt wurde in 40 ml abs. Methanol und 1 ml konz. Schwefelsäure gelöst und 19 h unter Rückfluß erhitzt. Das Lösungsmittell wurde im Vakuum entfernt und der Rückstand in Essigsäureethylester aufgenommen. Waschen mit gesättigter Natriumhydrogencarbonatlösung und Wasser, Trocknen über Natriumsulfat, Filtrieren und Entfernen des Lösungsmittels lieferten ein Öl, welches durch Flash-Chromatographie (Laufmittel: Pentan/Essigester 80:20) aufgereinigt wurde. Es wurden 0,77 g (2,4 mmol) 2-(4-Diethylamino-2-hydroxybenzoyl)-benzoesäuremethylester erhalten, deren Struktur NMR-spektroskopisch bestätigt wurde. λₘₐₓ: 354 nm; E¹₁: 1173.

### Beispiel 2

### Herstellung von (4-Diethylamino-2-hydroxyphenyl)-phenylketon

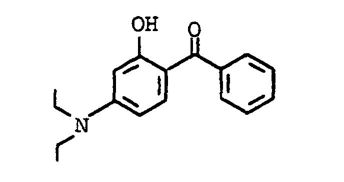

a)
   Eine Lösung von 2,99 g (18,1 mmol) 3-Diethylaminophenol, 2,7 ml (23,6 mmol) Benzoylchlorid und 2 ml Pyridin in 100 ml abs. Toluol wurde 3 h unter Rückfluß erhitzt. Nach Entfernen des Lösungsmittels im Vakuum wurde das Rohprodukt mittels Flash-Chromatographie (Laufmittel: Pentan/Essigester 90:10) gereinigt. Man erhielt 0,8 g Benzoesäure-(3-diethylamino)-phenylester.
b)

0,78 g (2,9 mmol) Benzoesäure-(3-diethylamino)-phenylester und 1,16 g (8,7 mmol) Aluminiumtrichlorid wurden gemischt und 4 h auf 175°C erhitzt. Nach dem Erkalten wurde die Reaktionsmischung mit Eiswasser und 2 ml konz. Salzsäure hydrolysiert. Es wurde 1 h bei Raumtemperatur gerührt, bevor die Reaktionsmischung mit 50 ml Dichlormethan versetzt wurde. Die organische Phase wurde abgetrennt und die wäßrige Phase zweimal mit 10 ml Dichlormethan extrahiert. Die vereinigten organischen Phasen wurden über Natriumsulfat getrocknet, filtriert und das Lösungsmittel im Vakuum entfernt. Das Rohprodukt wurde durch Flash-Chromatographie (Laufmittel: Pentan/Methyl-tert. Butylether 90:10) gereinigt. Man erhielt 0,22 g (4-Diethylamino-2-hydroxyphenyl)-phenylketon. Smp.: 46-48°C; λₘₐₓ: 359 nm; E¹₁: 1280.

### Beispiel 3

### Herstellung von 2-(2-Hydroxy-4-pyrrolidin-1-yl-benzol)-benzoesäure

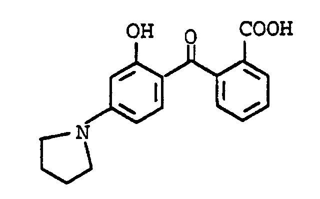

3,48 g (21,3 mmol) 3-Pyrrolidin-1-yl-phenol wurden in 60 ml abs. Toluol gelöst. Nach Zugabe von 3,47 g (23,4 mmol) Phthalsäureanhydid wurde die Reaktionsmischung 22 h unter Rückfluß erhitzt. Nach dem Erkalten wurde das Lösungsmittel im Vakuum entfernt und der Rückstand in 100 ml Chloroform aufgenonmmen. Die organische Phase wurde sechsmal mit 20 ml verdünnter Salzsäure und dann mit soviel Wasser gewaschen, daß die wäßrige Phase fast keinen Rhodaninfarbstoff mehr enthielt. Die organische Phase wurde sechsmal mit je 20 ml ges. Natriumhydrogencarbonatlösung extrahiert. Die vereinigten wäßrigen Extrakte wurden mit verd. Schwefelsäure angesäuert (pH < 2) und dreimal mit je 50 ml Ether extrahiert. Die vereinigten organischen Phasen wurden mit Wasser gewaschen, über Magesiumsulfat getrocknet, filtriert und das Lösungsmittel im Vakuum enfernt. Es wurden 4,22 g (13,5 mmol) 2-(2-Hydroxy-4-pyrrolidin-1-yl-benzol)-benzoesäure als Feststoff erhalten. Smp.: 203°C; λₘₐₓ: 355 nm; E¹₁: 1167.

### Beispiel 4

### Herstellung von 2-(2-Hydroxy-4-pyrrolidin-1-yl-benzol)-benzoesäuremethylester

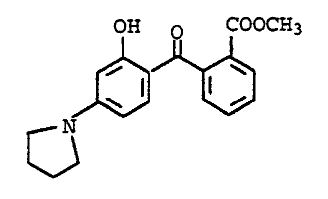

Zu einer Lösung von 1,32 g (4,2 mmol) 2-(2-Hydroxy-4-pyrrolidin-1-yl-benzol)-benzoesäure in 40 ml abs. Methanol wurde 1 ml konz. Schwefelsäure getropft. Die Reaktionsmischung wurde 16 h unter Rückfluß erhitzt. Das Lösungsmittel wurde im Vakuum entfernt. Der Rückstand wurde in 100 ml Essigsäureethylester aufgenommen, mit ges. Natriumhydrogencarbonatlösung und Wasser gewaschen, über Natriumsulfat getrocknet, filtriert und das Lösungsmittel im Vakuum entfernt. Das Rohprodukt wurde durch Flash-Chromatographie (Laufmittel: Pentan/Methyl-tert. Butylether 50:50) gereinigt. Man erhielt 1,14 g (3,5 mmol) 2-(2-Hydroxy-4-pyrrolidin-1-yl-benzol)-benzoesäuremethylester. Smp.: 164°C; λₘₐₓ: 355 nm; E¹₁: 1179.

Die Herstellung der Verbindungen 1 bis 8 der Tabelle 2 erfolgte analog den oben genannten Beispielen.

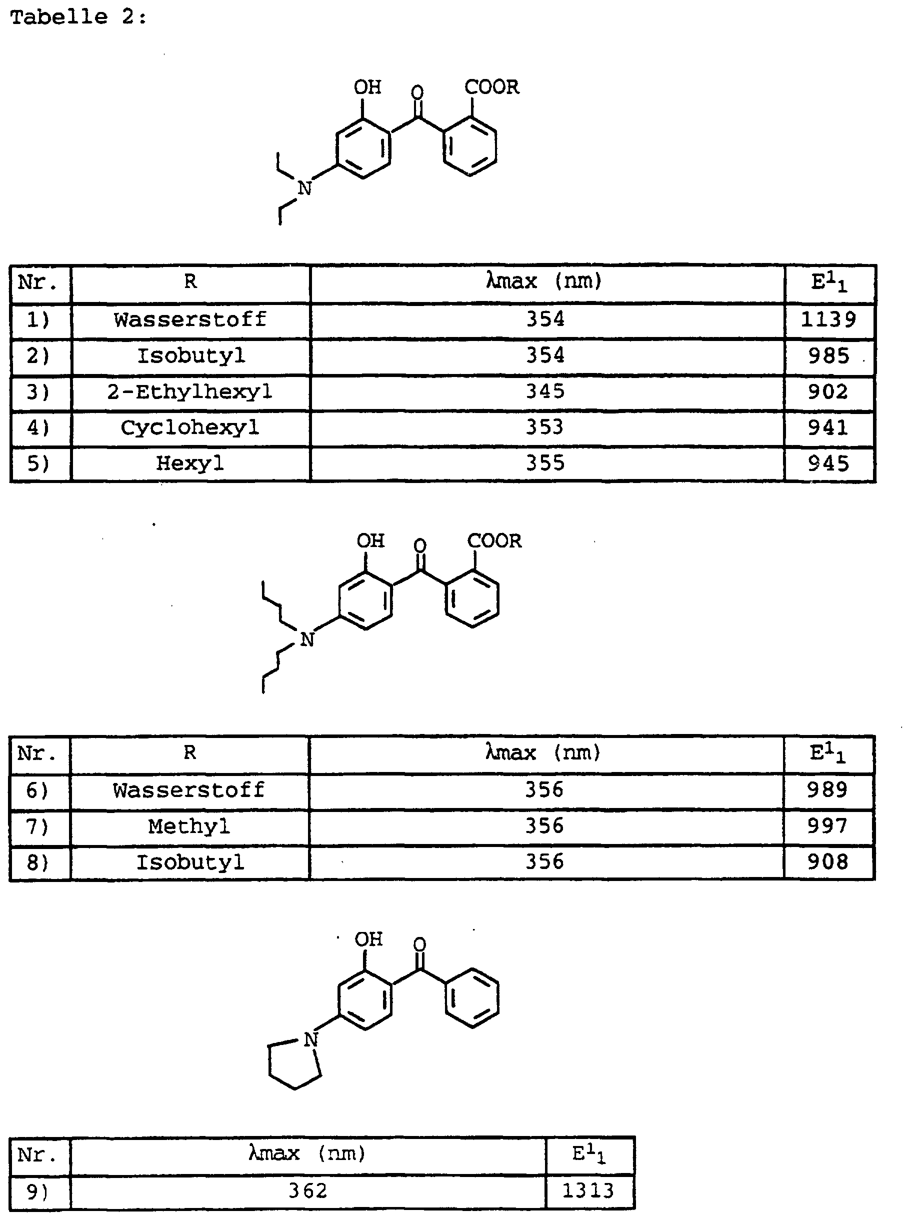

### Beispiel 5

### Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 6

### Zusammensetzung für die Lippenpflege

| Massengehalt | |
|---|---|
| (Gew.-%) | |
| ad 100 | Eucerinum anhydricum |
| 10,00 | Glycerin |
| 10,00 | Titanium Dioxid, mikronisiert |
| 5,00 | Verbindung Nr. 2 der Tabelle 2 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 7

### Zusammensetzung für die Lippenpflege

| Massengehalt | |
|---|---|
| (Gew.-%) | |
| ad 100 | Eucerinum anhydricum |
| 10,00 | Glycerin |
| 10,00 | Titanium Dioxid, mikronisiert |
| 5,00 | Verbindung Nr. 3 der Tabelle 2 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 8

### Zusammensetzung für Sunblocker mit Mikropigmenten

| Massengehalt | |
|---|---|
| (Gew.-%) | |
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Verbindung Nr. 2 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 9

### Zusammensetzung für Sunblocker mit Mikropigmenten

| Massengehalt | |
|---|---|
| (Gew.-%) | |
| ad 100 | Wasser |
| 10,00 | Octyl Methoxcinnamat |
| 6,00 | PEG-7-Hydrogenated Castor Öl |
| 6,00 | Titanium Dioxid, mikronisiert |
| 5,00 | Verbindung Nr. 3 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 10

### Fettfreies Gel

| Massengehalt | |
|---|---|
| (Gew.-%) | |
| ad 100 | Wasser |
| 8,00 | Octyl Methoxycinnamat |
| 7,00 | Titanium Dioxid, mikronisiert |
| 5,00 | Verbindung Nr. 2 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 11

### Fettfreies Gel

| Massengehalt | |
|---|---|
| (Gew.-%) | |
| ad 100 | Wasser |
| 8,00 | Octyl Methoxycinnamat |
| 7,00 | Titanium Dioxid, mikronisiert |
| 5,00 | Verbindung Nr. 3 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 12

### Sonnencreme (LSF 20)

| Massengehalt | |
|---|---|
| (Gew.-%) | |
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Verbindung Nr. 2 der Tabelle 2 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 13

### Sonnencreme (LSF 20)

| Massengehalt | |
|---|---|
| (Gew.-%) | |
| ad 100 | Wasser |
| 8,00 | Octyl Methoxycinnamat |
| 8,00 | Titanium Dioxid, mikronisiert |
| 6,00 | PEG-7-Hydrogenated Castor Öl |
| 5,00 | Verbindung Nr. 3 der Tabelle 2 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 14

### Sonnencreme wasserfest

| Massengehalt | |
|---|---|
| (Gew.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 2 der Tabelle 2 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 15

### Sonnencreme wasserfest

| Massengehalt | |
|---|---|
| (Gew.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 3 der Tabelle 2 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 16

### Sonnenmilch (LSF 6)

| Massengehalt | |
|---|---|
| (Gew.-%) | |
| ad 100 | Wasser |
| 10,00 | Mineral Öl |
| 6,00 | PEG-7-Hydrogenated Castor Öl |
| 5,00 | Isopropyl palmitat |
| 3,50 | Octyl Methoxycinnamat |
| 5,00 | Verbindung Nr. 2 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

### Beispiel 17

### Sonnenmilch (LSF 6)

| Massengehalt | |
|---|---|
| (Gew.-%) | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 3 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

## Patentansprüche

1. Verwendung von aminosubstituierten Hydroxybenzophenonen der allgemeinen Formel Ib, 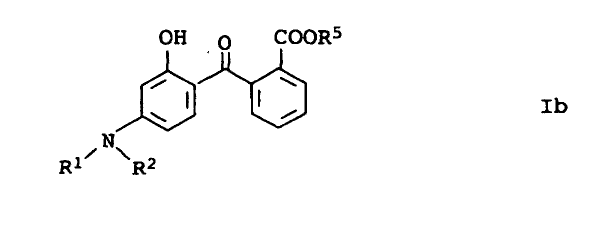 in der die Substituenten unabhängig voneinander folgende Bedeutung haben:
R¹ und R² Wasserstoff, C₁-C₁₂-Alkyl, wobei die Substituenten R¹ und R² gemeinsam mit dem Stickstoffatom, an dem sie gebunden sind, einen 5- oder 6-Ring bilden können;
R⁵ Wasserstoff, C₁-C₁₂-Alkyl, C₃-C₆-Cycloalkyl
als photostabile UV-A-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Verwendung von Verbindungen der Formel Ib nach Anspruch 1 als UV-Stabilisator in kosmetischen und pharmazeutischen Formulierungen.

3. Lichtschutzmittel enthaltende kosmetische und pharmazeutische Zubereitungen zum Schutz der menschlichen Epidermis oder menschlichen Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten im UV-Bereich absorbierenden Verbindungen, als photostabile UV-A-Filter wirksame Mengen von Verbindungen der Formel Ib 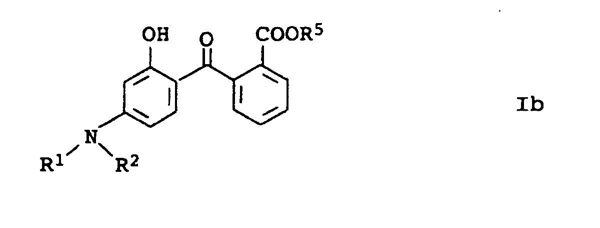 enthalten, in der die Variablen die Bedeutung definiert gemäß Anspruch 1 haben.

4. Aminosubstituierte Hydroxybenzophenone der allgemeinen Formel Ic, 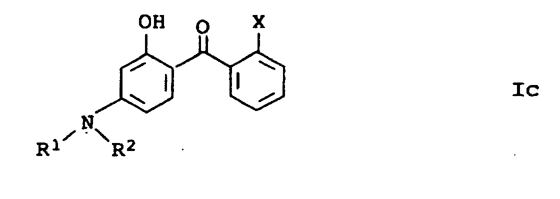 in der die Variablen unabhängig voneinander folgende Bedeutung haben:
R¹ und R² Wasserstoff, C₁-C₈-Alkyl, wobei die Substituenten R¹ und R² gemeinsam mit dem Stickstoffatom, an dem sie gebunden sind, einen 5- oder 6-Ring bilden können;
X COOR⁵, CONR⁶R⁷;
R⁵ C₂-C₁₂-Alkyl, C₅-C₆-Cycloalkyl;
R⁶ und R⁷ Wasserstoff, C₁-C₁₂-Alkyl, C₅-C₆-Cycloalkyl.

## Claims

1. The use of amino-substituted hydroxybenzophenones of the formula Ib, 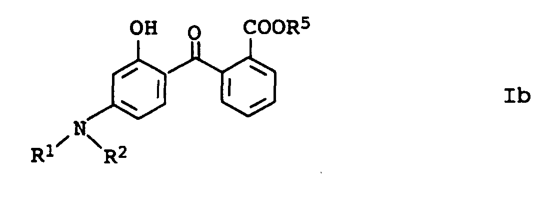 in which the substituents independently of one another have the following meanings:
R¹ and R² are hydrogen, C₁-C₁₂-alkyl, where the substituents R¹ and R² together with the nitrogen atom to which they are bonded can form a 5- or 6-membered ring;
R⁵ is hydrogen, C₁-C₁₂-alkyl, C₃-C₆-cycloalkyl
as photostable UV-A filters in cosmetic and pharmaceutical preparations for protecting the human skin or human hair from the sun's rays, alone or together with compounds which absorb in the UV region and are known per se for cosmetic and pharmaceutical preparations.

2. The use of compounds of the formula Ib as claimed in claim 1 as UV stabilizer in cosmetic and pharmaceutical formulations.

3. A sunscreen-containing cosmetic or pharmaceutical preparation for protecting the human epidermis or human hair from UV light in the range from 280 to 400 nm, which comprises, in a cosmetically and pharmaceutically suitable carrier, alone or together with compounds which absorb in the UV region and are known per se for cosmetic and pharmaceutical preparations, amounts, which are effective as photostable UV-A filters, of compounds of the formula Ib 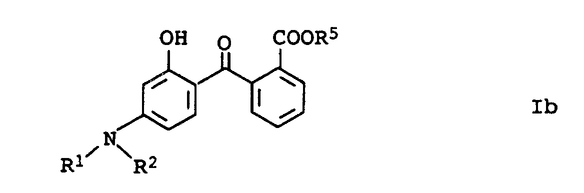 in which the variables have the meanings defined in claim 1.

4. An amino-substituted hydroxybenzophenone of the formula Ic, 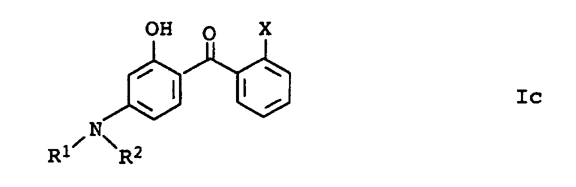 in which the variables independently of one another have the following meanings:
R¹ and R² are hydrogen, C₁-C₈-alkyl, where the substituents R¹ and R² together with the nitrogen atom to which they are bonded can form a 5- or 6-membered ring;
X is COOR⁵, CONR⁶R⁷;
R⁵ is C₂-C₁₂-alkyl, C₅-C₆-cycloalkyl;
R⁶ and R⁷ are hydrogen, C₁-C₁₂-alkyl, C₅-C₆-cycloalkyl.

## Revendications

1. Utilisation d'hydroxybenzophénones aminosubstituées de formule générale Ib, 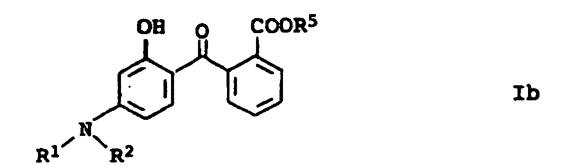 dans laquelle les substituants ont indépendamment l'un de l'autre la signification suivante:
R¹ et R² hydrogène, alkyle en C₁-C₁₂, tandis que les substituants R¹ et R² peuvent former conjointement avec l'atome d'azote auquel ils sont liés, un cycle à 5 ou 6 chaînons;
R⁵ hydrogène, alkyle en C₁-C₁₂, cycloalkyle en C₃-C₆,
comme filtre UV-A photostable dans des compositions cosmétiques et pharmaceutiques pour la protection de la peau humaine ou des cheveux humains contre le rayonnement solaire, seules ou conjointement avec des composés absorbant dans le domaine des UV, connus en soi pour des compositions cosmétiques et pharmaceutiques.

2. Utilisation des composés de formule Ib selon la revendication 1 comme stabilisant vis-à-vis des UV dans des formulations cosmétiques et pharmaceutiques.

3. Compositions cosmétiques et pharmaceutiques contenant des agents de protection contre la lumière, pour la protection de l'épiderme humain ou des cheveux humains, contre la lumière UV dans l'intervalle de 280 à 400 nm, **caractérisées par le fait qu'**elles contiennent, dans un support approprié du point de vue cosmétique et pharmaceutique, seuls ou conjointement avec des composés absorbant dans le domaine des UV, connus en soi pour des compositions cosmétiques et pharmaceutiques, à titre de filtre UV-A photostable, des quantités efficaces de composés de formule Ib 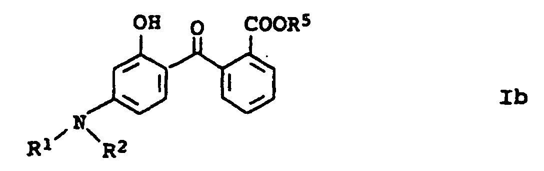 dans laquelle les variables ont la signification définie selon la revendication 1.

4. Hydroxybenzophénones aminosubstituées de formule générale Ic, 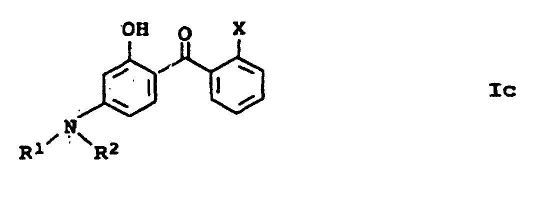 dans laquelle les variables ont indépendamment l'une de l'autre la signification suivante:
R¹ et R² hydrogène, alkyle en C₁-C₈, tandis que les substituants R¹ et R² peuvent former conjointement avec l'atome d'azote auquel ils sont liés, un cycle à 5 ou 6 chaînons;
X COOR⁵, CONR⁶R⁷;
R⁵ alkyle en C₂-C₁₂, cycloalkyle en C₅-C₆;
R⁶ et R⁷ hydrogène, alkyle en C₁-C₁₂, cycloalkyle en C₅-C₆.
